Europäisches Patentamt

(19) European Patent Office

Office européen des brevets

(11) Numéro de publication: **0 165 135**
**B1**

(12) **FASCICULE DE BREVET EUROPEEN**

(45) Date de publication du fascicule du brevet:
24.08.88

(21) Numéro de dépôt: 85400955.2

(22) Date de dépôt: 15.05.85

(51) Int. Cl.⁴: **C 07 C 51/15**, C 07 C 53/18,
C 07 C 53/21, C 07 C 145/00,
C 07 C 143/08

(54) Procédé de préparation d'acides trifluorométhylés.

(30) Priorité: 23.05.84 FR 8408010

(43) Date de publication de la demande:
18.12.85 Bulletin 85/51

(45) Mention de la délivrance du brevet:
24.08.88 Bulletin 88/34

(84) Etats contractants désignés:
AT BE CH DE FR GB IT LI LU NL SE

(56) Documents cités:
DE - A - 2 848 197
FR - A - 2 342 950
FR - A - 2 374 287

(73) Titulaire: RHONE-POULENC CHIMIE, 25, quai Paul
Doumer, F-92408 Courbevoie Cédex (FR)

(72) Inventeur: Wakselman, Claude, 18, rue du Clos
d'Alençon, F-91120 - Villebon-sur-Yvette (FR)
Inventeur: Tordeux, Marc, 23, rue des Ormeaux,
F-92260 Fontenay-aux-Roses (FR)

(74) Mandataire: Le Pennec, Magali et al, RHONE-POULENC
INTERSERVICES Service Brevets Chimie 25, Quai Paul
Doumer, F-92408 Courbevoie Cédex (FR)

## Description

La présente invention a trait à un procédé de préparation d'acides trifluorométhylés. Elle concerne plus particulièrement la préparation d'acide trifluoroacétique et d'acide trifluorométhanesulfonique.

La première méthode de synthèse de l'acide trifluorométhane sulfonique a été décrite par Haszeldine en 1954 [J. Chem. Soc. 4228 (1954)]. Elle consistait à faire une oxydation du di(trifluorométhylthio) mercure $(CF_3S)_2$ Hg par une solution aqueuse d'eau oxygénée en présence de rayonnement.

Cette méthode est absolument inexploitable industriellement, le sel de mercure n'étant pas disponible sur le marché et sa fabrication étant fort dangereuse car très toxique. D'autres procédés utilisant des intermédiaires tel que le bistrifluorométhyldisulfure $CF_3S-SCF_3$ ont aussi été décrits [J. Chem. Soc. 2901 (1955)], ces méthodes comme la précédente ne sont pas transposables à l'industrie à cause de la très forte toxicité du bistrifluorométhyldisulfure $CF_3S-SCF_3$.

La seule méthode industrielle de fabrication de l'acide trifluorométhane sulfonique est une méthode de fluoration électrochimique du chlorure de l'acide méthane sulfonique [J. Chem. Soc. 173 (1956)]. Les méthodes électrochimiques conduisent de par la difficulté de la technologie à la production d'acide dont le prix est extrêmement élevé.

Il est encore connu d'après le brevet US 4 221 734, un procédé de préparation d'acides carboxyliques perfluorés et d'acides sulfoniques perfluorés dans lesquels le radical alkyle contient au moins deux atomes de carbone par réaction d'halogénure de perfluoroalkyle et de $CO_2$ ou $SO_2$ dans un solvant en présence de zinc.

Les exemples décrits concernent uniquement la condensation des iodures de perfluoroalkyle avec soit $CO_2$ soit $SO_2$.

Un essai a été réalisé conformément à l'exemple 2 du brevet US 4 221 734 en utilisant du bromure de trifluorométhyle comme halogénure d'alkyle. Un très faible rendement en trifluorométhylsulfinate de zinc brut est obtenu, de l'ordre de 5% par rapport au zinc.

Ce brevet ne décrit pas un procédé industriel permettant la mise en œuvre d'un halogénure d'alkyle à un atome de carbone. La présente invention pallie les déficiences de l'art antérieur.

Il a maintenant été découvert un procédé de préparation d'acides trifluorométhylés caractérisé en ce que dans une première étape, on met en présence du dioxyde de carbone ou de soufre, un métal choisi parmi le zinc, l'aluminium, le manganèse, le cadmium, le magnésium, l'étain, le fer, le nickel et le cobalt dans un solvant aprotique polaire et dans une deuxième étape, on ajoute l'halogénure de trifluorométhyle, éventuellement en mélange avec le dioxyde de carbone et/ou de soufre, sous une pression supérieure à 1 bar.

Les acides trifluorométhylés obtenus par le procédé selon l'invention sont l'acide trifluoroacétique et l'acide trifluorométhane sulfonique.

On préfère parmi les métaux choisir ceux qui ne présentent qu'un seul degré d'oxydation et tout particulièrement ceux dont le potentiel d'oxydo réduction est compris entre –0,8 et –2 volts. Ainsi sont particulièrement préférés le zinc, l'aluminium, le manganèse et le cadmium.

Parmi ces derniers, d'un point de vue économique, il est avantageux de choisir le zinc et l'aluminium.

Le métal est avantageusement utilisé sous forme dispersée de façon à assurer le meilleur contact avec les gaz mis en œuvre dans le procédé de l'invention.

Le solvant choisi doit, autant que possible, permettre de solubiliser le dioxyde de carbone ou de soufre, l'halogénure de trifluorométhyle ainsi que les sels de l'acide formé. Répondent à cette condition, les solvants aprotiques polaires et parmi eux préférentiellement:

- l'acétonitrile
- le diméthylformamide (D.M.F.)
- le diméthylsulfoxide (D.M.S.O.)
- le diméthylacétamide (D.M.A.)
- l'hexaméthylphosphoramide (H.M.P.A.)
- la N-méthylpyrrolidone (N.M.P.)

Le diméthylformamide et le diméthylsulfoxide sont utilisés encore plus préférentiellement.

L'halogénure de trifluorométhyle utilisé est choisi parmi le bromure et l'iodure de trifluorométhyle. Parmi ces halogénures est particulièrement préféré le bromure de trifluorométhyle, moins onéreux, car c'est un produit industriel utilisé dans la lutte contre l'incendie. En effet, ce composé est réputé pour son inertie chimique et il est particulièrement surprenant qu'en présence de zinc et de dioxyde de carbone ou de soufre, il réagisse pour former soit un sel de zinc de l'acide trifluoroacétique si on utilise $CO_2$, soit un sel de zinc de l'acide trifluorométhane sulfinique si on utilise $SO_2$. Chacun de ces sels est déplacé de manière connue en soi par une base alcaline forte, choisie de préférence parmi la soude, la potasse, la chaux.

Le sel alcalin obtenu de l'acide trifluoroacétique est déplacé directement par l'acide sulfurique tandis que le sel de l'acide trifluorométhane sulfinique est d'abord oxydé en sel alcalin de l'acide trifluorométhane sulfonique avant d'être déplacé par l'acide sulfurique.

Selon un mode de mise en œuvre préféré de l'invention, on met en présence lors de la première étape, le zinc, l'anhydride d'acide métalloïdique et la base alcaline forte de préférence sous forme solide puis l'halogénure de trifluorométhyle.

Selon un mode encore plus préférentiel de l'invention, on opère en absence d'oxygène; pour cela, le première étape est effectuée sous vide ou sous atmosphère inerte.

Selon le procédé de l'invention, on met en œuvre préférentiellement un rapport molaire de l'halogénure de trifluorométhyle au métal d'au moins 2 et un rapport molaire du dioxyde de carbone ou de soufre au métal compris de préférence entre 1 et 3.

L'halogénure de trifluorométhyle en excès peut être recyclé.

La quantité de solvant mise en œuvre est telle qu'il y ait de préférence entre 0,25 et 1 atome gramme de métal par litre de solvant. Ces quantités ne sont pas essentielles, elles seront adaptées à l'économie du procédé.

La réaction étant exothermique, on maintient de préférence et seulement si cela est nécessaire la température entre 10 et 60°C.

La pression réactionnelle est au minimum de un bar; on utilise de préférence une pression comprise entre un et 50 bars, l'homme de l'art adaptera la pression à l'appareillage utilisé.

Il apparaît clairement à l'homme de l'art que le matériau constitutif du réacteur ne sera pas à base d'un des métaux utilisés dans le procédé de l'invention. Il est possible par exemple d'utiliser un réacteur en verre.

Les acides trifluorométhylés obtenus par le procédé de l'invention sont utilisés comme intermédiaires de synthèse dans l'industrie pharmaceutique ou phytosanitaire ou comme catalyseurs (Brevet japonais no 58 128 343).

L'invention va être plus complètement décrite à l'aide des exemples suivants qui ne doivent pas être considérés comme limitatifs de l'invention.

Exemple 1

Préparation du trifluorométhylsulfinate de sodium

Dans un flacon en verre épais, on place 300 ml de diméthylformamide et 5 g (0,077 mole) de zinc. Le flacon est placé dans un appareil de Parr. On fait le vide dans le flacon puis on ajoute 10 g (0,15 mole) de dioxyde de soufre puis 40 g (0,27 mole) de bromotrifluorométhane. La pression est alors de 2,6 bars. En quatre heures, sous agitation, elle redescend à 1,7 bar. On fait redescendre la pression à 0,6 bar, puis on ajoute 20 g de dioxyde de soufre; la pression est alors remontée à 1,4 bar. En deux heures, elle redescend à 1 bar. Le flacon est alors ouvert. Le diméthylformamide est distillé à 50°C/3000 Pa jusqu'à sec. On ajoute 300 ml d'eau puis de la soude en pastilles jusqu'à ce que le pH devienne légèrement basique. Les sels de Zn insolubles sont filtrés. L'eau est evaporée sous vide et le solide est extrait à l'acétate d'éthyle. Après évaporation de l'acétate d'éthyle, on obtient 8 g de solide brut. Ce solide contient 66% de trifluorométhylsulfinate de sodium ($0_F$/-87 ppm). Le rendement de la transformation du zinc en trifluorométhylsulfinate de sodium est donc de 44% par rapport au Zn. Une autre expérience sans deuxième addition de dioxyde de soufre a conduit à un rendement de 37%.

Transformation en trifluorométhylsulfonate de sodium

8 g de trifluorométhylsulfinate de sodium bruts sont dissous dans 25 ml d'eau oxygénée à 35%.

Après 5 heures, on constate par PMN du $^{19}$F que la transformation est totale. Il reste un seul signal à $0_F$:-78 ppm. On ajoute alors 2 grains de pierre ponce. Le mélange est porté à ébullition. L'eau oxygénée est décomposée pour donner 2,3 litres d'oxygène. L'eau est évaporée et le solide est extrait à l'acétone pour donner après évaporation 7,2 g de trifluorométhylsulfonate de sodium brut.

Transformation en acide trifluorométhanesulfonique

3 g de trifluorométhylsulfonate de sodium et 3 g d'acide sulfurique à 95% sont portés à 140°C sous un vide de 100 Pa. On recueille 2,25 g de monohydrate d'acide trifluorométhanesulfonique (PF: 45°C). Ces 2,25 g sont neutralisés avec 12,6 ml de soude normale et on obtient, après séchage à 80°C/60 Pa, 2,3 g de trifluorométhylsulfonate de sodium. PF: 251°C, $0_F$:-78 ppm.

Exemple 2

Préparation du trifluorométhylsulfinate de sodium

Dans un flacon en verre épais, on place 100 ml de diméthylformamide, 3,5 g (0,054 moles) de zinc en poudre et 4 g d'hydroxyde de sodium en poudre. Le flacon est placé dans un appareil de Parr. On fait le vide. On ajoute 15 g de dioxyde de soufre puis 20 g (0,13 mole) de bromotrifluorométhane. La pression est alors de 2,7 bars. En deux heures, sous agitation, la pression est redescendue à 0,7 bar. Le flacon est alors ouvert, les solides sont séparés par filtration; le diméthylformamide est distillé à 50°C sous 3000 Pa. On ajoute alors 200 ml d'eau, le pH est ramené à 7 par addition de NaOH. Après décantation, l'eau est evaporée sous pression réduite; le résidu est repris à l'acétate d'éthyle. Les solides sont éliminés par filtration et l'acétate d'éthyle évaporé sous pression réduite.

Transformation en trifluorométhane sulfonate de baryum

On ajoute 20 ml d'eau oxygénée à 35%. On agite pendant 17 heures puis le mélange est porté à 100°C avec une pierre à ébullition jusqu'à ce qu'il n'y ait plus de dégagement d'oxygène. L'eau est evaporée sous vide, on verse 10 ml d'acide sulfurique à 98% puis l'acide trifluorométhanesulfonique est distillé rapidement sous 100 Pa par chauffage à 130°C. Le distillat est dissous dans 100 ml d'eau. On ajoute de l'hydroxyde de baryum jusqu'à pH7. Après filtration, l'eau est évaporée sous vide. Le résidu est repris à l'acétone. Après évaporation de l'acétone, on obtient 6,4 g (0,015 mole) de trifluorométhane sulfonate de baryum. Rdt 55% par rapport au zinc.

Exemple 3

Préparation de l'acide trifluoroacétique

3,25 g de zinc, 50 ml de diméthylformamide et 12 g de neige carbonique sont placés dans un flacon en verre épais. La pression dans le flacon disposé dans un appareil de Parr est abaissée jusqu'à 1 bar par dégagement de $CO_2$. La pression

est ensuite augmentée jusqu'à 2,7 bars par adjonction de bromotrifluorométhane. Sous agitation pendant 3 heures, la pression redescend à 0,8 bar. Le flacon est alors ouvert. Le diméthylformamide est distillé à 50°C/3000 Pa. Après traitement par la soude et extraction à l'acétate d'éthyle, on ajoute 5 ml d'acide sulfurique à 95% au solide obtenu. Le mélange est porté à 145°C et on recueille par distillation 1,15 g d'acide trifluoroacétique. (Rdt 20% par rapport au zinc.)

## Exemple 4

Préparation de l'acide trifluorométhane sulfonique

Dans un flacon en verre épais, on place 100 ml de diméthylformamide, 1,5 g (0,055 mole) d'aluminium en poudre. Le flacon est placé dans un appareil de Parr. On fait le vide puis on ajoute 15 g de dioxyde de soufre et 20 g (0,13 mole) de bromotrifluorométhane. La pression est alors de 2,7 bars. En deux heures, sous agitation, la pression est redescendue à 1,4 bars. Le flacon est alors ouvert, les solides sont séparés par filtration; le diméthylformamide est distillé à 50°C sous 3000 Pa. On ajoute alors 200 ml d'eau, le pH est ramené à 7 par addition de soude. Après décantation, l'eau est évaporée sous pression réduite; le résidu est repris à l'acétate d'éthyle. Les solides sont éliminés par filtration. L'acétate d'éthyle est évaporé sous pression réduite. On ajoute 20 ml d'eau oxygénée à 35%; on agite pendant 17 heures puis le mélange est porté à 100°C avec une pierre à ébullition jusqu'à ce qu'il n'y ait plus de dégagement d'oxygène. L'eau est évaporée sous vide, on verse 10 ml d'acide sulfurique à 98% puis l'acide trifluorométhanesulfonique est distillé rapidement à 130°C sous 100 Pa. On obtient 3,9 g (0,022 mole) d'acide trifluorométhane sulfonique monohydrate (Rdt: 40% par rapport à l'aluminium).

## Exemple 5

Préparation de l'acide trifluorométhanesulfonique

Dans un flacon en verre épais, on place 100 ml de diméthylformamide, 3 g (0,055 mole) de manganèse en poudre. Le flacon est placé dans un appareil de Parr. On fait le vide puis on ajoute 15 g de dioxyde de soufre et 20 g (0,13 mole) de bromotrifluorométhane. La pression est alors de 2,7 bars. En deux heures, sous agitation, la pression est redescendue à 1,6 bar. Le flacon est alors ouvert, les solides sont séparés par filtration; le diméthylformamide est distillé à 50°C sous 3000 Pa. On ajoute alors 200 ml d'eau, le pH est ramené à 7 par addition de soude. Après décantation, l'eau est évaporée sous pression réduite; le résidu est repris à l'acétate d'éthyle. Les solides sont éliminés par filtration, l'acétate d'éthyle évaporé sous pression réduite. On ajoute 20 ml d'eau oxygénée à 35%. On agite pendant 17 heures puis le mélange est porté à 100°C avec une pierre à ébullition jusqu'à ce qu'il n'y ait plus de dégagement d'oxygène. L'eau est évaporée sous vide, on verse 10 ml d'acide sulfurique à 98% puis l'acide trifluorométhanesulfonique est distillé rapidement à 130°C sous 100 Pa. On obtient 2,5 g (0,014 mole) d'acide trifluorométhanesulfonique monohydrate (Rdt: 25% par rapport au manganèse).

## Exemple 6

Préparation de l'acide trifluorométhanesulfonique

Dans un flacon en verre épais, on place 100 ml de diméthylformamide, 3 g (0,054 mole) de cadmium en poudre. Le flacon est placé dans un appareil de Parr. On fait le vide puis on ajoute 15 g de dioxyde de soufre et 20 g (0,13 mole) de bromotrifluorométhane. La pression est alors de 2,7 bars. En deux heures, sous agitation, la pression est redescendue à 1 bar. Le flacon est alors ouvert, les solides sont séparés par filtration; le diméthylformamide est distillé à 50°C sous 3000 Pa. On ajoute alors 200 ml d'eau, le pH est ramené à 7 par addition de soude. Après décantation, l'eau est évaporée sous pression réduite; le résidu est repris à l'acétate d'éthyle. Les solides sont éliminés par filtration, l'acétate d'éthyle évaporé sous pression réduite. On ajoute 20 ml d'eau oxygénée à 35%. On agite pendant 17 heures puis le mélange est porté à 100°C avec une pierre à ébullition jusqu'à ce qu'il n'y ait plus de dégagement d'oxygène. L'eau est évaporée sous vide, on verse 10 ml d'acide sulfurique à 98% puis l'acide trifluorométhanesulfonique est distillé rapidement à 130°C sous 100 Pa. On obtient ainsi 4,4 g (0,025 mole) d'acide trifluorométhane sulfonique monohydrate (Rdt: 45% par rapport au cadmium).

## Exemple 7

Préparation de l'acide trifluoroacétique

3,25 g de zinc, 50 ml de diméthylformamide et 12 g de neige carbonique sont placés dans un flacon en verre épais. La pression dans le flacon disposé dans un appareil de Parr est abaissée jusqu'à 1 bar par dégagement de $CO_2$. La pression est ensuite augmentée jusqu'à 2,7 bars par adjonction de bromotrifluorométhane contenant 1% en volume de $SO_2$. Sous agitation pendant 2 heures, la pression redescend à 2 bars. On ajoute à nouveau du bromotrifluorométhane contenant 1% de $SO_2$ jusqu'à 2,7 bars. On attend une heure, la pression redescend à 2,2 bars. Le flacon est alors ouvert. Le diméthylformamide est distillé à 50°C/3000 Pa. Après traitement par la soude et extraction à l'acétate d'éthyle, on ajoute 5 ml d'acide sulfurique à 95% au solide obtenu. Le mélange est porté à 145°C et on recueille par distillation 1,70 g d'acide trifluoroacétique. (Rdt: 30% par rapport au zinc).

## Exemple 8

Préparation du trifluorométhylsulfinate de sodium

Dans un flacon en verre épais, on place 100 ml de diméthylformamide, 3,5 g (0,054 mole) de zinc en poudre et 5,5 g (0,055 mole) de métabisulfite de sodium anhydre en poudre. Le flacon est placé dans un appareil de Parr. On fait le vide. On ajoute 15 g de dioxyde de soufre puis 25 g (0,17 mole) de bromotrifluorométhane. La pression est alors de 3,2 bars. En deux heures, sous agitation, la pression est redescendue à 0,7 bar. Le flacon est alors ouvert, les solides sont séparés par filtration; le diméthylformamide est distillé à 50°C sous 3000 Pa. On ajoute alors 200 ml d'eau, le pH est ramené à 7 par addition de NaOH. Après décantation, l'eau est évaporée sous pression réduite; le résidu est repris à l'acétate d'éthyle. Les solides sont éliminés par filtration et l'acétate d'éthyle évaporé sous pression réduite.

Transformation en trifluorométhane sulfonate de baryum

On ajoute 20 ml d'eau oxygénée à 35%. On agite pendant 17 heures puis le mélange est porté à 100°C avec une pierre à ébullition jusqu'à ce qu'il n'y ait plus de dégagement d'oxygène. L'eau est évaporée sous vide, on verse 10 ml d'acide sulfurique à 98% puis l'acide trifluorométhanesulfonique est distillé rapidement sous 100 Pa par chauffage à 130°C. Le distillat est dissous dans 100 ml d'eau. On ajoute de l'hydroxyde de baryum jusqu'à pH 7. Après filtration, l'eau est évaporée sous vide. Le résidu est repris à l'acétone. Après évaporation de l'acétone, on obtient 7,9 g (0,018 mole) de trifluorométhane sulfonate de baryum. Rdt: 68% par rapport au zinc.

## Revendications

1. Procédé de préparation d'acide trifluoroacétique et d'acide trifluorométhane sulfonique caractérisé en ce que dans une première étape, on met en présence du dioxyde de carbone ou de soufre, un métal choisi parmi le zinc, l'aluminium, le manganèse, le cadmium, le magnésium, l'étain, le fer, le nickel et le cobalt dans un solvant aprotique polaire, dans une deuxième étape, on ajoute l'halogénure de trifluorométhyle éventuellement en mélange avec le dioxyde de carbone et/ou de soufre sous une pression supérieure à 1 bar.

2. Procédé selon la revendication 1 caractérisé en ce que l'halogénure de trifluorométhyle est le bromure de trifluorométhyle.

3. Procédé selon la revendication 1 caractérisé en ce que le métal est choisi parmi le zinc, l'aluminium, le manganèse et le cadmium.

4. Procédé selon la revendication 1 et la revendication 4 caractérisé en ce que le métal est choisi parmi le zinc et l'aluminium.

5. Procédé selon la revendication 1 caractérisé en ce que le solvant aprotique polaire est choisi parmi le diméthylformamide, le diméthylsulfoxide, l'acétonitrile, l'hexaméthylphosphoramide, le diméthylacétamide, la N-méthylpyrrolidone.

6. Procédé selon la revendication 1 caractérisé en ce que le rapport molaire de l'halogénure de trifluorométhyle au métal est d'au moins 2.

7. Procédé selon la revendication 1 caractérisé en ce que le rapport molaire de l'anhydride d'acide au métal est compris entre 1 et 3.

8. Procédé selon la revendication 1 caractérisé en ce que la quantité de solvant est telle qu'on ait entre 0,25 et 1 atome gramme de métal par litre de solvant.

9. Procédé selon la revendication 1 caractérisé en ce que la pression réactionnelle est comprise entre 1 et 50 bars.

10. Procédé selon l'une quelconque des revendications précédentes caractérisé en ce que, au cours de la première étape, on ajoute une base alcaline forte ou un métabisulfite alcalin.

## Patentansprüche

1. Verfahren zur Herstellung von Trifluoressigsäure und Trifluormethansulfonsäure, dadurch gekennzeichnet, dass man in einer ersten Verfahrensstufe Kohlendioxid oder Schwefeldioxid und ein unter Zink, Aluminium, Mangan, Cadmium, Magnesium, Zinn, Eisen, Nickel und Cobalt ausgewähltes Metall in einem aprotischen polaren Lösungsmittel vorlegt und in einer zweiten Verfahrensstufe das Trifluormethylhalogenid, gegebenenfalls im Gemisch mit dem Kohlendioxid und/oder Schwefeldioxid unter einem Druck von mehr als 1 bar zugibt.

2. Verfahren nach Anspruch 1, dadurch gekennzeichnet, dass das Trifluormethylhalogenid Trifluorbrommethan ist.

3. Verfahren nach Anspruch 1, dadurch gekennzeichnet, dass das Metall unter Zink, Aluminium, Magnesium und Cadmium ausgewählt wird.

4. Verfahren nach den Ansprüchen 1 und 3, dadurch gekennzeichnet, dass das Metall unter Zink und Aluminium ausgewählt wird.

5. Verfahren nach Anspruch 1, dadurch gekennzeichnet, dass das polare aprotische Lösemittel unter Dimethylformamid, Dimethylsulfoxid, Acetonitril, Hexamethylphosphorsäureamid, Dimethylacetamid und N-Methylpyrrolidon ausgewählt wird.

6. Verfahren nach Anspruch 1, dadurch gekennzeichnet, dass das Molverhältnis von Trifluormethylhalogenid zu Metall wenigstens 2 beträgt.

7. Verfahren nach Anspruch 1, dadurch gekennzeichnet, dass das Molverhältnis des Säureanhydrids zu Metall zwischen 1 und 3 beträgt.

8. Verfahren nach Anspruch 1, dadurch gekennzeichnet, dass die Menge des Lösungsmittels derart ist, dass zwischen 0,25 und 1 g Atom Metall pro Liter Lösungsmittel vorhanden sind.

9. Verfahren nach Anspruch 1, dadurch gekennzeichnet, dass der Druck während der Reaktion zwischen 1 und 50 bar liegt.

10. Verfahren nach einem der vorhergehenden Ansprüche, dadurch gekennzeichnet, dass man im ersten Schritt eine starke Alkalibase oder ein Alkalimetabisulfit zugibt.

## Claims

1. Process for the preparation of trifluoroacetic acid and of trifluoromethanesulphonic acid, characterized in that, in a first stage, carbon dioxide or sulphur dioxide is placed in contact with a metal chosen from zinc, aluminium, manganese, cadmium, magnesium, tin, iron, nickel and cobalt in a polar aprotic solvent and, in a second stage, the trifluoromethyl halide is added optionally mixed with carbon dioxide and/or sulphur dioxide at a pressure of more than 1 bar.

2. Process according to Claim 1, characterized in that the trifluoromethyl halide is trifluoromethylbromide.

3. Process according to Claim 1, characterized in that the metal is chosen from zinc, aluminium, manganese and cadmium.

4. Process according to Claim 1 and Claim 4, characterized in that the metal is chosen from zinc and aluminium.

5. Process according to Claim 1, characterized in that the polar aprotic solvent is chosen from dimethylformamide, dimethyl sulphoxide, acetonitrile, hexamethylphosphoramide, dimethylacetamide and N-methylpyrrolidone.

6. Process according to Claim 1, characterized in that the molar ration of the trifluoromethyl halide to the metal is at least 2.

7. Process according to Claim 1, characterized in that the molar ratio of the acid anhydride to the metal is between 1 and 3.

8. Process according to Claim 1, characterized in that the quantity of solvent is such that there is between 0.25 and 1 gram-atom of metal per litre of solvent.

9. Process according to Claim 1, characterized in that the reaction pressure is between 1 and 50 bars.

10. Process according to any one of the preceding claims, characterized in that, during the first stage, a strong alkaline base or an alkali metal metabisulphite is added.